# EUROPEAN PATENT SPECIFICATION

(11) **EP 2 214 705 B1**
(45) Date of publication and mention of the grant of the patent: **20.08.2014**
(21) Application number: 08856104.8
(22) Date of filing: 20.11.2008
(51) Int. Cl.: A61K 39/12, A61K 39/385, A61K 39/145, A61K 39/29, A61K 39/39, C07K 16/08, A61K 39/00

(54) **A POWERFUL VACCINE COMPOSITION COMPRISING A LIPOPEPTIDE AND POLY I:C AS AN ADJUVANT**
WIRKUNGSVOLLE IMPFSTOFFZUSAMMENSETZUNG MIT EINEM LIPOPEPTID UND POLY-I:C ALS WIRKSTOFF
COMPOSITION PUISSANTE D'UN VACCIN COMPRENANT UN LIPOPEPTIDE ET POLY I:C COMME ADJUVANT

(30) Priority: 07.12.2007 KR 20070126775
(43) Date of publication of application: 11.08.2010
(73) Proprietor: Dobeel Co., Ltd., Seongnam-si, Kyonggi-do 462-716 (KR)
(72) Inventor: MOON, Hong Mo, GwangJu-si Gyeonggi-do 464-120 (KR); AHN, Byung Cheol, Yongin-si Gyeonggi-do 448-542 (KR); YUM, Jung Sun, Seongnam-si Kyonggi-do 463-914 (KR)
(74) Representative: Office Freylinger
(86) International application number: PCT/KR2008/006842
(87) International publication number: WO 2009/072767

(56) References cited:
- WO-A2-2006/040076
- JP-A- 2007 077 073
- KR-A- 20070 029 111
- US-A1- 2007 160 632
- WARGER TOBIAS ET AL: "Synergistic activation of dendritic cells by combined Toll-like receptor ligation induces superior CTL responses in vivo.", BLOOD 15 JUL 2006 LNKD- PUBMED:16537810, vol. 108, no. 2, 15 July 2006 (2006-07-15) , pages 544-550, XP000002658391, ISSN: 0006-4971
- LUHRMANN ET AL: "Improved intranasal immunization with live-attenuated measles virus after co-inoculation of the lipopeptide MALP-2", VACCINE, ELSEVIER LTD, GB, vol. 23, no. 39, 15 September 2005 (2005-09-15), pages 4721-4726, XP005040629, ISSN: 0264-410X, DOI: 10.1016/J.VACCINE.2005.05.009
- ASAHI-OZAKI Y ET AL: "Intranasal administration of adjuvant-combined recombinant influenza virus HA vaccine protects mice from the lethal H5N1 virus infection", MICROBES AND INFECTION, ELSEVIER, PARIS, FR, vol. 8, no. 12-13, 1 October 2006 (2006-10-01), pages 2706-2714, XP025243408, ISSN: 1286-4579, DOI: 10.1016/J.MICINF.2006.07.018 [retrieved on 2006-10-01]
- BAGCHI ARANYA ET AL: "MyD88-dependent and MyD88-independent pathways in synergy, priming, and tolerance between TLR agonists.", JOURNAL OF IMMUNOLOGY (BALTIMORE, MD. : 1950) 15 JAN 2007 LNKD- PUBMED:17202381, vol. 178, no. 2, 15 January 2007 (2007-01-15), pages 1164-1171, XP000002658393, ISSN: 0022-1767
- LAHIRI A ET AL: "Engagement of TLR signaling as adjuvant: Towards smarter vaccine and beyond", VACCINE, ELSEVIER LTD, GB, vol. 26, no. 52, 9 December 2008 (2008-12-09), pages 6777-6783, XP025711304, ISSN: 0264-410X, DOI: 10.1016/J.VACCINE.2008.09.045 [retrieved on 2008-11-27]
- SPOHN, R. ET AL.: 'Synthetic lipopeptide adjuvants and Toll-like receptor 2-structure-activity relationships' VACCINE vol. 22, no. 19, 23 June 2004, pages 2494 - 2499, XP004515469

## Description

### Technical Field

The present invention relates to a vaccine, a therapeutic agent, an immunological agent comprising a lipopeptide selected from the group consisting of Pam3Cys-SKKKK and Pam3Cys-SR8; and poly I:C (polyinosinic:polycytidylic acid).

### Background Art

An adjuvant plays a role in promoting immune response by accelerating or amplifying one or more specific phases of various immune response inducing processes. Therefore, when an adjuvant is co-administered with an antigen, it can improve immunogenicity of the antigen and/or alters the type of immune response against the antigen. Typical examples of such adjuvant are oil emulsion (Freund's adjuvant), monophosphoryl lipid A (MPL), Q saponins, aluminum hydroxide or phosphate or calcium salts (alum) of aluminum, non-ionic block polymer surfactants, lipopolysaccharides, mycobacteria, tetanus toxoid, CpG, etc.

Use of a protein antigen alone does not often induce strong enough immune response and a desired type of immune response, so the vaccine composition contains normally antigen and an adjuvant. According to two signal model for immune responses, a signal delivered through the engagement of antigen epitope presented with MHC molecule and antigen receptor is not enough to induce an immune response. It requires additional signals generated from costimulatory molecule(s). In this the adjuvant may be able to enforce signal strength generated by costimulatory molecules and/or induce costimulatory molecules and also induce cytokines that determine the type of immune responses. Some antigens such as lipoproteins, glycoproteins, or whole microorganisms can provide both epitopes and adjuvant function in the form of pathogen associated molecular pattern (PAMP). The primary structure of protein antigens, that is, the amino acid sequence of an antigen cannot be changed, but the PAMP of an antigen can be modified or supplemented by the addition of a proper adjuvant or subsidiary structure to affect immunogenicity (Dempsey PW et al., Science 271: 348-350, 1996; Deres K et al., Nature 342: 561-564, 1989). The modification of a molecular pattern of an antigen can increase immunogenicity and also affect the type of elicited immune response. For example, in the case of HBV surface antigen, S-protein without preS1 and preS2 does not exhibit immunogenicity in certain congenic mouse strains, while L-protein containing preS1 and preS2 not only induces antibody generation against preS1 and preS2 but also helps to induce antibody generation against S antigen (Milich DR et al., 1986. New Approaches to Immunization, pp 377-382. Cold Spring Harbor Laboratories, New York). In the case when a whole pathogenic microorganism is used as an antigen, it is expected for the microorganism to contain various types of PAMP such as lipopolysaccharides, nucleic acids, lipoproteins and conjugated proteins. In this situation, pathogen recognition receptor (PRR) existing on the surface of antigen presenting cell(APC) recognizes PAMP to generate signals to induce various costimulatory molecules and cytokines, which affects the type of immune response as well as the magnitude. For example, interferon gamma and IL-12 helps to induce Th1 (T helper cell 1) response playing an important role in immune response against virus infection. Th1 type immune response leads to the increase of IgG2a and IgG2b generation and induce powerful cell mediated immune response. In this, various types of PAMPs associated with antigens are playing as an adjuvant functions and such adjuvant can help in regulating immune responses. However, often, these types of natural adjuvant function associated with the antigens may not be strong enough to induce desired strength and quality of immune responses, requiring a good adjuvant in vaccine formulation. Lührmann et al describe that the macrophage-activating lipopeptide activates presenting cells of human, mouse and rat origin *in vitro* and *in vivo* ("Improved intranasal immunization with live-attenuated measles virus co-inoculation of the lipopeptide MALP-2" Vaccine 23 (2005) 4721-4726).

Therefore, developing a good adjuvant is a very important job in developing a good vaccine, but an adjuvant development still has to be relied mainly on empirical work. For example, Toll Like Receptors (TLR) are the most important PRR on antigen presenting cells (APC) involved in the activation of APC and in antigen presentation by APC. Potent antibody response, however, is not entirely dependent on TLR signals (Gavin. A. L. et al, Science 314:1936-1938, 2006). Further, Pam3cys, which is one of TLR2 ligands, works in inducing immune response independently of TLR2 (Yoder et al, Infect. Immun. 71:3894-3900, 2003). To make matters further complicated, good protective immune response requires balanced immune response comprising both strong cell mediated immune response and humoral antibody response. Therefore, developing an adjuvant that will help to induce well balanced adaptive immune response is still beyond the matter that can be rationally predicted.

In this invention, inventors have defined a powerful vaccine as a vaccine formulation that can generate a large amount of high quality antigen specific antibody in reference to the most well known adjuvant aluminum hydroxide. The generation of an appropriate, high quality antibody is a very important factor for producing a good preventive or an effective therapeutic vaccine. For example, IgG isotypes play different roles in elimination of a tumor cells; IgG2a is the most effective one, compared with IgG1, IgG2b or IgG3 (Nimmerjahn F & Ravetch JV, Science 310: 1510-1512, 2005). IgG2a and IgG2b, known to be the most effective in inducing antiviral immunity (Coutelier JP et al., J Exp Med 165:64-69, 1987; Markine-Gorianoff D & Coutelier JP, J of Virol 76:432-435, 2002), are generated by cytokines produced by Th1 cells, which also induce cell mediated immune response. Therefore, the induction of Th1 cell response is a good indication for the generation of an appropriate, high quality antibody. The most widely utilized adjuvant, Alum, induces Th 2 type immune response, and induced antibody is mainly IgG1.

Thus, the powerful vaccine composition of the present invention is judged by the amount of an antigen specific antibody generated and high ratios of IgG2a/IgG1 and IgG2b/IgGl comparing to widely utilized Alum adjuvant containing vaccine. The adjuvant described in this invention is the one that is able to induce powerful antibody response as well as cell-mediated immune response and that can switch immunoglobulin isotype to produce IgG2a and IgG2b.

Present inventors completed this invention by confirming that an adjuvant composition comprising lipopeptide and poly I:C (polyinosinic:polycytidylic acid) is far more powerful than the conventional adjuvant, aluminum hydroxide, and that further confirming lipopeptide and Poly I:C are synergistic instead of additive in stimulation of adaptive immune responses. Although Pam3Cys and poly I:C is known to be synergistic in inducing TNF-α and IL-6 in macrophage (Bagchi,A.et al, J. Immun. 178:1164-1171, 2007), well balanced powerful adjuvant function of a similar combination consisting of a lipopeptide and poly I:C is an unexpected finding. Present inventors confirmed further that covalent linking of lipopeptide to antigen is not required. Simple formulation in the form of a mixture of a lipopeptide, poly I:C, and at least one antigen is sufficient.

### Disclosure

### Technical objectives

The present document describes an adjuvant that can help antigen to induce strong immune response.

It is another objective of the present invention to provide a vaccine composition containing the above adjuvant and an antigen.

The present document describes a method for generating an appropriate, high quality antibody using the above adjuvant.

The present document describes a method for enhancing Th1 immune response using the aforementioned adjuvant.

It is also an objective of the present invention to provide a therapeutic vaccine for viral or parasite infection, containing aforementioned adjuvant and at least one viral or parasite antigen.

It is also an objective of the present invention to provide a preventive or therapeutic vaccine against cancer, containing the adjuvant and at least one cancer-specific antigen.

### Technical Solution

To achieve above objectives, the present document describes an adjuvant comprising a lipopeptide selected from the group consisting of Pam3Cys-SKKKK and Pam3Cys-SR8; and poly I:C (polyinosinic:polytidylic acid).

The present invention also provides a vaccine composition containing the above adjuvant and at least one viral or parasite antigen for use in the treatment of viral or parasitic infection.

The present invention also provides a therapeutic agent comprising the above adjuvant and at least one viral or parasite antigen for use in the treatment of viral or parasitic infection.

The present invention also provides an immunological agent comprising the above adjuvant and at least one cancer-specific antigen for use in the prevention or the treatment of cancer.

The present document describes a method for generating an appropriate, high quality antibody comprising steps of administrating the above vaccine composition to a subject in need thereof.

The present document describes a method for enhancing Th1 immune response comprising steps of administrating the above vaccine composition to a subject in need thereof.

The present invention also provides a therapeutic vaccine against viral or parasite infection, comprising the adjuvant composition and at least one viral or parasite antigen.

The present invention also provides a prophylactic or therapeutic vaccine against cancer, containing the adjuvant composition and at least one cancer-specific antigen.

The present document describes the use of the adjuvant and at least one viral or parasite antigen in the manufacture of a therapeutic agent for treating viral or parasite infection.

In addition, the present document describes the use of the adjuvant and at least one cancer-specific antigen in the manufacture of a therapeutic agent for treating cancer.

### Advantageous Effect

When the adjuvant of the present invention is used together with an antigen, the antigen specific antibody induction is stimulated and Th1 type immune response is also induced. Therefore, the adjuvant of the present invention is very effective as an adjuvant for vaccine formulation for the prevention and treatment of viral or parasite infection or cancer.

### Description of Drawings

The application of the preferred embodiments of the present invention is best understood with reference to the accompanying drawings, wherein:
Figure 1 is a graph showing the titer of S-protein antibody elicited by various vaccine formulations containing L-HBsAg composed of L-protein (S-protein-preS2-preSl), M-protein (S-protein-preS2), and S-protein in aluminum hydroxide (Alum), Pam3Cys-SKKKK alone, poly I:C alone, or both Pam3Cys-SKKKK and poy I:C as adjuvants.
Figure 2 is a graph showing the titer of antibody against preS antigen induced by various vaccine formulations with L-HBsAg and Alum, Pam3Cys-SKKKK alone, poly I:C alone, or both Pam3Cys-SKKKK and Poly I:C as adjuvants. In this experiment pronounced effect of synergy between Pam3Cys-SKKKK and poly I:C can be seen.
Figure 3 is a set of graphs showing the antibody isotypes induced by various different vaccine formulations with L-HBsAg in Alum, Pam3Cys-SKKKK alone, poly I:C alone, or both Pam3Cys-SKKKK and Poly I:C as adjuvants.
   a; Antibody titer of each isotype, b; IgG2a/IgG1,
   c; IgG2b/IgG1.
Figure 4 is a graph showing the immunogenicity against Influenza virus HA antigen formulated with different adjuvants; Alum, Pam3Cys-SKKKK alone, poly I:C alone, or both Pam3Cys-SKKKK and Poly I:C.
Figure 5 is a set of graphs showing the antibody isotypes induced by Influenza virus antigen formulated with different types of adjuvants; Alum, Pam3Cys-SKKKK alone, poly I:C alone, or both Pam3Cys-SKKKK and Poly I:C.
   a; Antibody titer of each isotype
   b; IgG2a/IgG1
Figure 6 is a graph showing the immunogenicity against HBsAg antigen (S-protein) from *Hansenula polymorpha* and preS from *Saccharomyces cereviciae* formulated in different adjuvants; Alum, Pam3Cys-SKKKK alone, poly I:C alone, or both Pam3Cys-SKKKK.
Figure 7 is a graph showing the immunogenicity against preS antigen by vaccines formulated with *Hansenula* S-protein and preS from yeast in different adjuvants; Alum, Pam3Cys-SKKKK alone, poly I:C alone, or both Pam3Cys-SKKKK and Poly I:C.
Figure 8 is a set of graphs showing the antibody isotypes induced by vaccines formulated with HBsAg (S-protein) from *Hansenula* and preS from yeast in different adjuvants; Alum, Pam3Cys-SKKKK alone, poly I:C alone or both Pam3Cys-SKKKK.
   a; Antibody titer of each isotype, b; IgG2a/IgG1,
   c; IgG2b/IgG1.
Figure 9 is a graph showing the titer of S-protein antibody elicited by various vaccine formulations containing L-HBsAg composed of L-protein (S-protein-preS2-preS1), M-protein (S-protein-preS2), and S-protein in aluminum hydroxide (Alum), Pam3Cys-SKKKK alone, Pam3Cys-SR8 alone, FSL-1 alone, poly I:C alone, combination of Pam3Cys-SKKKK and poy I:C, combination of Pam3Cys-SR8 and poy I:C, or combination of FSL-1 and poy I:C as adjuvants.
Figure 10 is a graph showing the titer of antibody against preS antigen induced by various vaccine formulations with L-HBsAg and aluminum hydroxide (Alum), Pam3Cys-SKKKK alone, Pam3Cys-SR8 alone, FSL-1 alone, poly I:C alone, combination of Pam3Cys-SKKKK and poy I:C, combination of Pam3Cys-SR8 and poy I:C, or combination of FSL-1 and poy I:C as adjuvants.
   In this experiment pronounced effect of synergy between lipopeptide and poly I:C can be seen.
Figure 11 is a set of graphs showing the antibody isotypes induced by various different vaccine formulations with L-HBsAg in aluminum hydroxide (Alum), Pam3Cys-SKKKK alone, Pam3Cys-SR8 alone, FSL-1 alone, poly I:C alone, combination of Pam3Cys-SKKKK and poy I:C, combination of Pam3Cys-SR8 and poy I:C, or combination of FSL-1 and poy I:C as adjuvants.
   a; Antibody titer of each isotype, b; IgG2a/IgG1,
   c; IgG2b/IgG1.

### Best Mode

Hereinafter, the present invention is described in detail.

The present document describes an adjuvant for vaccine, comprising one or more lipopeptides and poly I:C (polyinosinic:polytidylic acid).

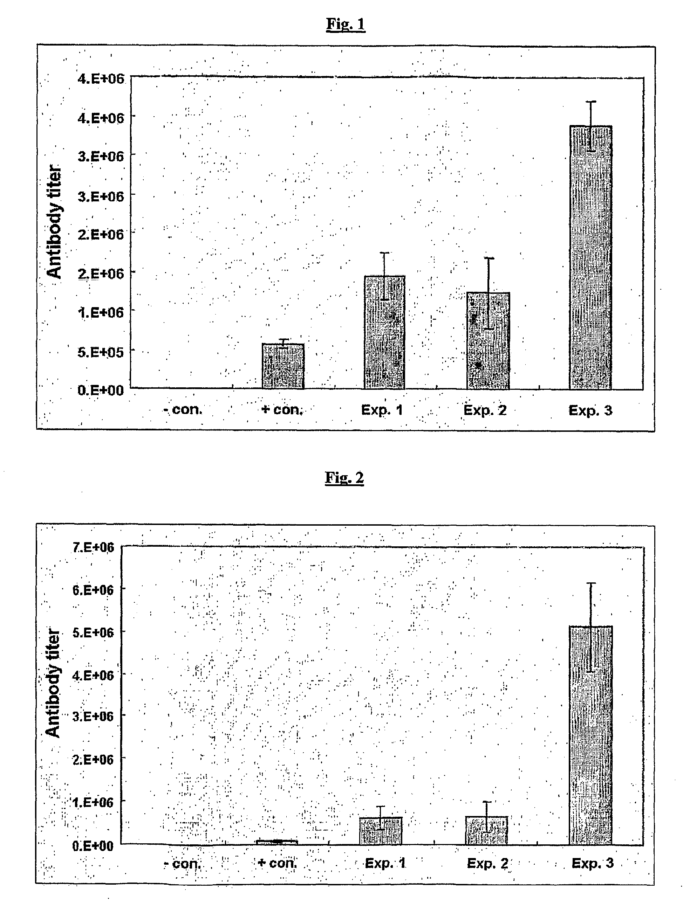

In a preferred embodiment of the present invention, Pam3Cys-SKKKK, a kind of lipopeptides, and poly I:C were mixed and this mixture was used as an adjuvant to produce a vaccine with L-HBsAg, influenza antigen, or a mixture of HBsAg S-protein and PreS as antigens. And the vaccine containing mixture of Pam3Cys-SKKKK and Poly I:C was confirmed to enhance the antigen-specific antibody production significantly, compared to most frequently used conventional adjuvant aluminum hydroxide (see Figures 1, 2, 4, 6 and 7, and Tables 1,2 and 3).

When the mixture of Pam3Cys-SKKKK and Poly I:C was used, it was synergistic in stimulating immune responses; that is, the titer of pre S antibody induced by the mixuture was several times more than the combined value of the antibody titer induced by individual components of the mixture. This is exemplified best in the figure 2, in which the antibody titer of pre S antibody was assessed for the mixture or individual components. This synergistic effect is less pronounced for S-protein antibody induction (Fig.1). This is due to the amount of antigen used in all expriments was saturating amount instead of right amount that will show adjuvant dependency. Pre S content in L-protein is less than 10% of the total (5 mg).

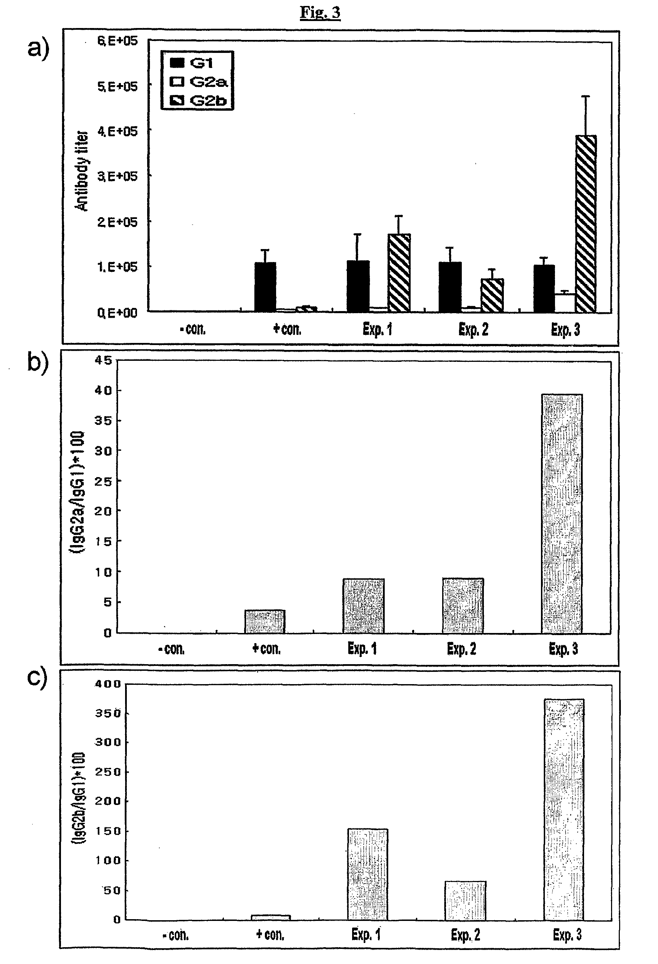

When a vaccine was formulated using aluminum hydroxide, which is known to induce Th2 immune response, IgG1 antibody was predominant IgG isotype. Whereas, when a vaccine was formulated using the mixture comprising Pam3Cys-SKKKK and poly I:C, IgG2a and IgG2b were produced dominantly. Therefore, the ratios of IgG2a/IgG1 and IgG2b/IgG1 were higher with the mixture of Pam3Cys-SKKKK and Poly I:C compare to the ratio obtained with conventional adjuvant aluminum hydroxide (see Figures 3, 5 and 8, and Tables 1, 2 and 3). This increase of IgG2a and IgG2b known to be very effective in defense against viral infection and cancer suggests the improvement of quality of immune response with the new adjuvant. This findings indicate that the adjuvant comprising lipopeptide and poly I:C, the present invention, can be effectively used for the development of powerful therapeutic and prophylactic vaccine formulations.

The lipopeptide was first synthesized by Metzger *et al.* as a synthetic analogue of lipopeptide originated from bacteria and mycoplasma (Metzger J et al., Int J Peptide Protein Res 37:46-57, 1991). Since then, numbers of analogues have been synthesized (EMC microcollections GmbH Sindelfinger Str. 372070 Tubingen, Germany). There is a report that virus-specific cytotoxic T lymphocyte (CTL) was induced by administrating a mouse with Pam3Cys-Ser-Ser, a kind of lipopeptides conjugated with influenza virus T cell epitope (Schild H et al, Eur J Immunol 21:2649-2654, 1991). In general, lipopeptide has been known as a TLR2 ligand (Trinchieri G & Sher A, Nat Rev Immunol 7:179-190, 2007).

In this invention, the lipopeptide is composed of fatty acids linked to glycerol and amino acids. Lipopeptides contain one or more fatty acids in each molecule. The lipopeptide can be a lipoprotein composed of a part of or a whole molecule originated from gram positive or gram negative bacteria or mycoplasma. And, the fatty acid and amino acid herein can be synthesized with chemical modifications. The lipopeptide herein is exemplified by Pam3Cys-SKKKK (formula 1; molecular structure: N-palmitoyl-S-[2,3-bis(palmitoyloxy)-(2RS)-propyl]-[R]-cystein-SKKKK), PHC-SKKKK, O1e2PamCys-SKKKK, Pam2Cys-SKKKK, PamCys(Pam)-SKKKK, O1e2Cys-SKKKK, Myr2Cys-SKKKK, PamDhc-SKKKK, PamCSKKKK and Dhc-SKKKK, but not always limited thereto.

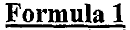

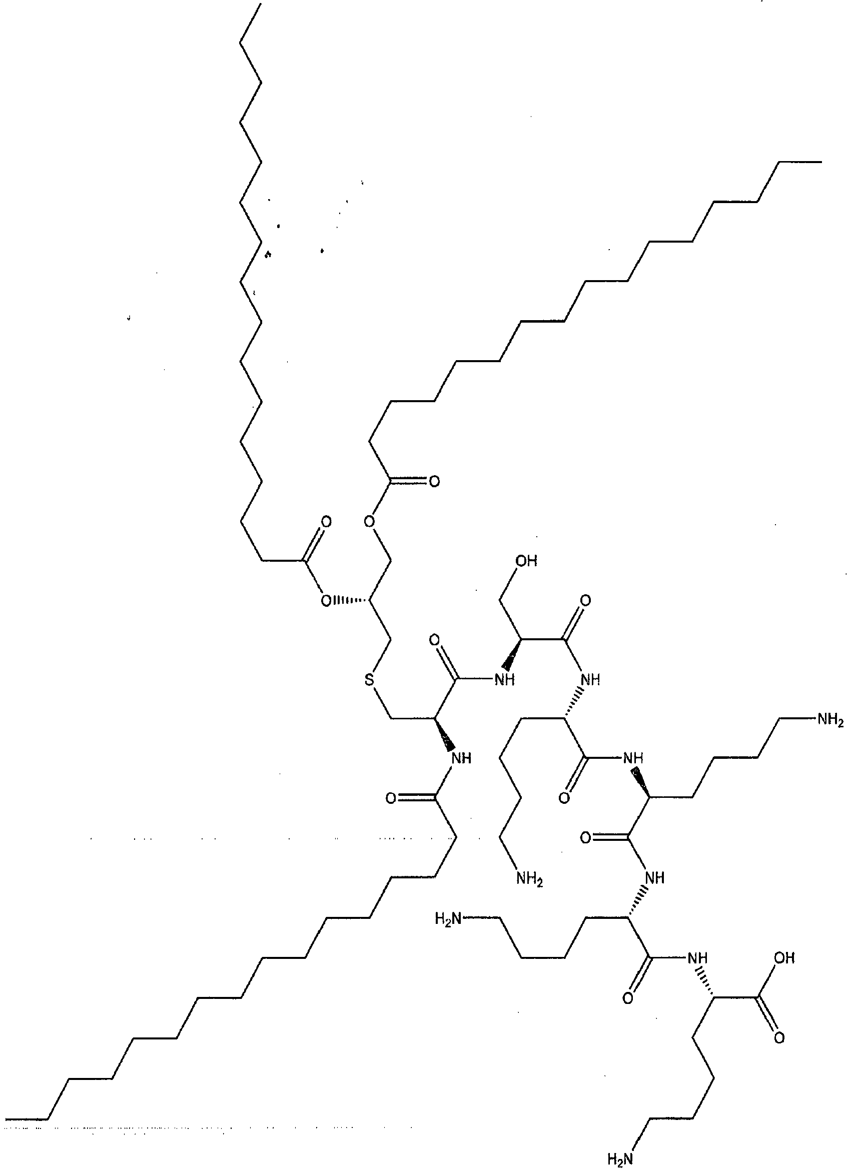

Poly I:C has been used as a powerful inducer of type I interferon in *in vitro* and *in vivo* studies (Magee ME & Griffith MJ, life Science II, 11:1081-1086, 1972; Manetti YR et al., Eur J Immunol 25:2656-2660, 1995), and has been known to induce dendritic cell (DC) maturation, most popular antigen presenting cell (APC) in mammals. Mature DC is capable of inducing immune response effectively (Rous R et a.l., International Immunol 16:767-773, 2004). Poly I:C is also known as an IL-12 inducer, and the IL-12 is an important cytokine inducing cell mediated immune response and IgG2a antibody generation by promoting the enhancement of Th1 development. Adjuvant activity of poly I:C was also shown previously (Cui Z & Qui F, Cancer Immunol Immunotherapy 16:1-13, 2005).

The poly I:C (polyinosinic:polycytidylic acid) is a synthetic double stranded RNA, and the length is preferably 50 - 2000 bp and more preferably 100 - 500 bp.

The present invention provides a vaccine composition containing said adjuvant comprising a lipopeptide selected from the group consisting of Pam3Cys-SKKKK and Pam3Cys-SR8; and poly I:C and at least one viral or parasite antigen for use in the treatment of viral or parasitic infection. It is also disclosed an adjuvant comprising one or more lipopeptides and poly I:C that can stimulate immune response synergistically instead of giving additive effect by each adjuvant component.

In a preferred embodiment of the present invention, a vaccine prepared by using the adjuvant above-described was proved to increase synergistically antigen specific antibody production as well as changing the quality of immune response, inducing mostly IgG2a and IgG2b (see Figures 1 - 8 and Tables 1 - 3). Therefore, an adjuvant composition containing said adjuvant components as above-described can be effectively used to increase immunogenicity of antigen and thereby improving the efficacy of vaccine.

The antigen herein indicates any material that can induce immune response by the immune system of animal or human. It can be a full length or a fragment. The antigen can be provided as synthetic material, purified subunits, a whole microbe or a mixture, but purified antigen is preferred.

The antigen herein is exemplified by a recombinant protein, or peptide from hepatitis virus or viral protein from influenza virus, but it can be a polysaccharide, a glycoprotein, a lipopolysaccharide, a DNA molecule, a cancer cell, a live virus or a hapten molecule, etc. The protein of a pathogen above is exemplified by influenza virus antigen (HA: haemagglutinin or neuraminidase antigen), but it can be *Bordetella pertussis* antigen, pertussis toxin, filamentous haemagglutinin, human papilloma virus (HPV) antigen, *Helicobacter pylori* antigen (capsular polysaccharides of serogroup A, B, C, Y and W-135), tetanus toxoid, diphtheria antigen (diphtheria toxoid), pneumococcal antigen (*Streptococcus pnemoniae* type 3 capsular polysaccharides), tuberculosis antigen, human immunodeficiency virus (HIV) antigen (GP-120, GP-160), cholera antigen (cholera toxin B subunit), staphylococcal antigen (staphylococcal enterotoxin B), shigella antigen (shigella polysaccharides), vesicular stomatitis virus antigen (vesicular stomatitis virus glycoprotein), cytomegalovirus (CMV) antigen, hepatitis antigen [hepatitis A(HAV), B(HBV), C(HCV), D(HDV) and G(HGV): L-HBsAg, S-HBsAg, M-HBsAg, pre S], respiratory synctytial virus (RSV) antigen, herpes simplex antigen and combinations thereof (ex: diphtheria, pertussis and tetanus; DPT), but not always limited thereto.

The vaccine composition of the present invention can additionally include, in addition to the adjuvant and an antigen, one or more effective ingredients having the same or similar effect with them. The vaccine composition can also include, in addition to the above-mentioned effective ingredients, one or more pharmaceutically acceptable carriers for the administration. The pharmaceutically acceptable carrier can be selected or be prepared by mixing more than one ingredients selected from a group consisting of saline, sterilized water, Ringer's solution, buffered saline, dextrose solution, maltodextrose solution, glycerol and ethanol. Other general additives such as anti-oxidative agent, buffer solution, bacteriostatic agent, etc., can be added. In order to prepare injectable solutions such as aqueous solution, suspension and emulsion, diluents, dispersing agents, surfactants, binders and lubricants can be additionally added. The vaccine composition of the present invention can further be prepared in suitable forms for each disease or according to ingredients by following a method represented in Remington's Pharmaceutical Science (the newest edition, Mack Publishing Company, Easton, PA).

The vaccine composition of the present invention can be administered parenterally by various routs such as subcutaneous injection, intravenous injection, intramuscular injection and intrathoracic injection. To prepare the vaccine composition as a formulation for parenteral administration, the vaccine composition is mixed with a stabilizer or a buffering agent to produce a solution or suspension, which is then formulated as a content of ampoules, syringes, or vials in single or multiple doses.

The vaccine composition of the present invention can be formulated in various forms according to the administration pathway. For example, the vaccine composition can be prepared as a sterilized aqueous solution or suspension for injection or a freeze-dried form. The freeze-dried vaccine composition is stored typically at 4°C and can be restored with a stabilizer containing with or without an additive such as saline and/or HEPES.

The dosage of the vaccine composition of the present invention can be determined by considering administration method and frequency, or disease under treatment, severity of disease, history of disease, co-treatment with another therapeutic agent or not, and age, height, weight, health condition, or physical condition, but not always limited thereto. In general, the dose of this vaccine composition is preferably increased according to the weight increase of a patient under treatment.

The present document describes a method for generating an appropriate, high quality antibody comprising the step of administering the above vaccine composition to a subject.

In a preferred embodiment of the present invention, the vaccine composition prepared by using the adjuvant as previously described increased antigen specific antibody production and IgG2a and IgG2b production (see Figures 1 - 10 and Tables 1 - 4). Therefore, the vaccine composition of the present invention can be effectively used for the mass-production of an appropriate, high quality antibody in which stimulation of immunogenicity of antigen is required.

In a preferred embodiment of the present invention, the vaccine composition can be administered parenterally, by intraperitoneal injection, hypodermic injection, intravenous injection or intramuscular injection. The vaccine composition can be administered at the dose enough to stimulate immune response in a subject. For example, the vaccine can be administered to human once or several times, each time at the dose of 1 - 250 µg and more preferably 10 - 100 µg.

The present document also discloses a method for enhancing Th1 immune response, comprising procedures of administering the above vaccine composition to a subject in need thereof.

In a preferred embodiment of the present invention, the vaccine composition prepared by using the adjuvant of the present invention increased IgG2a and IgG2b production (see Figures 3, 5, 8 and 10, and Tables 1, 2, 3 and 4). Therefore, the vaccine composition of the present invention can be effectively used for enhancing Th1 immune response to improve immunogenicity

The present invention also provides an immune therapeutic agent for viral or parasite infection, containing the said adjuvant comprising one or more lipopeptide selected from the group consisting of Pam3Cys-SKKKK and Pam3Cys-SR8; and polyI:C and at least one viral or parasite antigen composition as active ingredients for use in the treatment of viral or parasitic infection.

The present inventionalso provides use of the adjuvant and at least one viral or parasite antigen in the manufacture of a therapeutic agent for treating viral or parasite infection.

In a preferred embodiment of the present invention, the therapeutic agent composition prepared by using the adjuvant of the present invention increased antigen specific antibody production and IgG2a and IgG2b production (see Figures 1 - 10 and Tables 1 - 4). IgG2a and IgG2b are known to be effective in defending viral infection and cellular infection of parasites. Therefore, the vaccine composition comprising the adjuvant for vaccine of the present invention and at least one viral or parasite antigen can be effectively used as a therapeutic agent for viral or parasite infection.

In a preferred embodiment of the present invention, the viral antigen is influenza virus antigen (HA: haemagglutinin or neuraminidase antigen), human papilloma virus (HPV) antigen, human immunodeficiency virus (HIV) antigen (GP-120, GP-160), vesicular stomatitis virus antigen (vesicular stomatitis virus glycoprotein), cytomegalovirus (CMV) antigen, hepatitis antigen [hepatitis A(HAV), B(HBV), C(HCV), D(HDV) and G(HGV): L-HBsAg, S-HBsAg, M-HBsAg, pre S], respiratory synctytial virus (RSV) antigen or herpes simplex virus antigen.

In preferred embodiment of the present invention, the parasite is protozoa, nematoda, trematoda or cestoda but not limited thereto.

The protozoa is preferrably a rhizopoda, a mastigophora, a ciliate or a sporozoa but not limited thereto.

The rhizopoda includes *Entamoeba histoytica* and *Entamoeba coli.*

The mastigophora includes *Giardia lamblia, Trichomonas vaginalis, Trichomonas hominis* and *Haemoflagellates.*

The cilliate includes *Balantidium coli.*

The sporozoa is preferably a *Plasmodium sp.* including *P. vivax* and *P. falciparum,* a *Toxoplasma gondii, Pneumocystis carinii, Isospora hominis, Cryptosporidium sp.* including *C. parvum* and *C*. *muris.*

The nematoda is preferrably a whipworm, a hookworm, a pinworm, an ascarid or a filariodea but not limited thereto.

The whipworm is preferably *Trichuris trichiura* or *Trichocephalus trichiuris* but not limited thereto. However, any other whipworms infecting animals such as dogs, cats and pigs may be included.

The hookworm is preferably *Ancylostoma duodenale* or *Necator americanus* but not limited thereto.

The pinworm is preferably *Enterobius vermicularis* or *Enterobius gregorii.*

The ascarid is preferably *A*. *suum* which typically infects pigs or *A*. *lumbricoides* which infects humans.

The filariodea is preferably *Wuchereria bancrofti, Onchocerca volvulus, Loa loa* or *Dirofilaria immitis.*

The trematoda is preferably Digenea, but not limited thereto. The Digenea includes Schistosome or non-Schistosome.

The schistosome includes *Schistosoma mansoni, Schistosoma haematobium, Schistosoma japonicun* and *Schistosoma intercalatum,* but not limited thereto.

The non-Schistosome includes *Fasciolopsis buski, Heterophyes heterophyes, Metagonimus yokogawaii, Gastrodiscoides hominis, Clonorchis sinensis, Fasciola hepatica* and *Paragonimus westermani,* but not limited thereto.

The cestoda is preferably Taeniidae or Diphyllobothriidae but not limited thereto.

The Taeniidae includes Taenia solium and Taenia saginata. The Diphyllobothriidae includes Diphyllobothrium sp. such as *Diphyllobothrium latum, Diphyllobothrium dendriticum, D. nihonkaiense, D. pacificum, D. cordatum, D. ursi, D. lanceolatum, D. dalliae,* and *D. yonagoensis.*

The "parasite antigen" means a molecule derived from a parasite which is capable of inducing humoral response in a host. The parasite antigen can be a surface glyco- protein or a carbohydrate molecule thereof or a lipid molecule.

With respect to particular parasite antigens, the art discloses various parasite antigens.

Particularly, WO9526402A1 discloses a helminth parasite antigen characterized by (i) in native form being an integral membrane protein; (ii) having a native localization in the parasite gut; (iii) being capable of binding to a thiol affinity medium; and (iv) being recognised by sera from immunised animal hosts. WO9512671A1 discloses a helminth parasite antigen possesing aminopeptidase-like activity. US6399077 discloses a noninfectious soluble fraction of a *Toxoplasma gondii* infected cell culture lysate. US6413521 discloses an isolated and purified antigen conferring protective immunity against a non-obligate blood feeding helminth and which is characterized by possessing aminopeptidase M-like activity. US20070087012A1 discloses a novel Fasciclin Related Adhesive Protein (FRAP) from Plasmodium and related parasites. WO9402169A1 discloses a protective metazoan parasite antigen capable of binding to pepstatin. US4656251 discloses parasite antigens of *Dirofilaria immitis.* US4839275 circulating parasite antigens of *Dirofilaria immitis.*

The therapeutic agent against viral or parasite infection can be administered parenterally by hypodermic injection, intravenous injection or intramuscular injection. To prepare the vaccine composition as a formulation for parenteral administration, the vaccine composition of the present invention is prepared as an oil emulsion, which is then formulated as ampoules, syringes or vials. The effective dosage can be determined according to absorption rate, inactivation rate, age, gender, health condition of a patient, and severity of disease, etc.

The present invention also provides a preventive or therapeutic agent for cancer containing the adjuvant comprising one or more lipopeptide selected form the group consisting of Pam3Cys-SKKKK and Pam3Cys-SR8; and polyI:C and at least one cancer-specific antigen composition as active ingredients.
The cancer is preferably a renal cell carcinoma, a melanoma, a chronic lymphocytic leukemia, a lung cancer, a cervical cancer, a stomach cancer, a thyroid cancer, a pancreatic cancer, a breast cancer, a prostate cancer, an ovarian cancer, a cholangioma, a liver cancer, a colon cancer, or a rectal cancer, but not limited thereto.

The "cancer-specific antigen" is a protein or an immunologically active fragment thereof which is differentially expressed in cancerous tissues rather than normal tissues. Various cancer-specific antigens are known in the art. For example, gp100, MART-1 and MAGE-1 are well-known antigen specific for menanoma. The other cancer-specific antigen includes tyrosinase, CEA (cancer embryonic antigen), PSA (prostate specific antigen), HER2/neu, MAGE-1, MAGE-2, MAGE-3, NY-ESO-1, MUC-1, SART-1 or SART-3, TERT (telomerase reverse transcriptase) or partial peptides derived from TERT, WT1 or partial peptides derived from WT1, Survivin-2B or partial peptides derived from Survivin-2B, gp75, MDM2, telomerase, alpha-1 fetoprotein, CA125, CA15-3, CA19-9, G250 and NY-ESO-1, but not limited thereto (See WO 2006/078059 and WO 2007/065957).

Additional cancer-associated antigen (CAA) and a method for identifying CAA is disclosed in Miller (Drug Discovery Today, 8: 31-38, 2003) and Kawakami and Rosenberg (Immunol. Res. 16:313, 2003) and Slingluff et al. (Curr. Opin. Immunol., 6:733, 1994).

In addition, the present invention provides use of the adjuvant as a vaccine composition in the manufacture of an immunological therapeutic agent for treating cancer, in that strong cellular immune response is required.

In a preferred embodiment of the present invention, the immunological therapeutic agent composition prepared by using the adjuvant of the present invention increased antigen-specific antibody production and IgG2a and IgG2b production (see Figures 1 - 10 and Tables 1 - 4). IgG2a and IgG2b are known to be very effective in anticancer immune response. Therefore, the immunological therapeutic agent composition containing the adjuvant for vaccine of the present invention and an appropriate cancer-specific antigen can be effectively used as a preventive or therapeutic agent for preventing or treating cancer.

The immunological agent for preventing or treating cancer can be administered parenterally by hypodermic injection, intravenous injection or intramuscular injection. To prepare the vaccine composition as a formulation for parenteral administration, the vaccine composition of the present invention is formulated as an oil emulsion, which is then stored as ampoules, syringes, or vials. The effective dosage can be determined according to absorption rate, inactivation rate, age, gender, health condition of a patient, and severity of disease, etc.

### Mode for Invention

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Stimulation of immunogenicity of hepatitis B virus (HBV) antigen

Vaccines were prepared with hepatitis B virus antigen and various adjuvants including aluminum hydroxide (Alum; Brenntag Biosector, Germany), Pam3Cys-SKKKK (lipopeptide) (EMC microcollections GmbH, Germany) alone, poly I:C (Sigma, USA) alone, or the mixture of both Pam3Cys-SKKKK and poly I:C, and the induced antibody titer of each vaccine formulation was compared.

### <1-1> Preparation of vaccines and administration

Vaccines were prepared by mixing hepatitis B virus whole surface antigen (L-HBsAg; Korean Patent No: 10-0836745) and the said adjuvant, which were administered to mice. In this, L-HBsAg is consisted of S-protein (small protein without pre S1 and pre S2), M-protein (medium protein with pre S2 only), and L-protein (large protein with both preS1 and preS2).

Particularly, as shown in Table 1, 20 µg of Pam3Cys-SKKKK or poly I:C or both Pam3Cys-SKKKK and poly I:C were mixed with 0.5 µg of L-HBsAg to give a vaccine in oil emulsion form and induced antibody titer by different formulations was compared. As a positive control, the same amount of antigen was formulated with aluminum hydroxide. Vaccines were injected intra muscularly into 6 weeks old C57BL/6 female mice three times at two weeks intervals. The negative control was injected with PBS without antigen and adjuvant.

**Table 1**

| | Negative control | Positive control | Experimental group 1 | Experimental group 2 | Experimental group 3 |
|---|---|---|---|---|---|
| Adjuvant | - | Aluminum hydroxide | Pam3Cys-SK KKK | Poly I:C | Pam3Cys-SK KKK + poly I:C |
| HBsAg antibody titer | - | 5.7 × 10⁵ | 1.4 × 10⁶ | 1.2 × 10⁶ | 3.4 × 10⁶ |
| PreS antibody titer | - | 7.1×10⁴ | 6.2×10⁵ | 6.5×10⁵ | 5.1×10⁶ |
| IgG2a/IgG1 (%) | - | 3.6 | 8.9 | 9.1 | 39.4 |
| IgG2b/IgG1 (%) | - | 9.5 | 153.9 | 66.4 | 374.8 |

### <1-2> Analysis of immune response

### <1-2-1> Antibody titer against HBsAg (S-protein)

Serum was collected before the vaccine administration and 2 weeks after the third vaccine administration and antigen specific antibody generation was analyzed by ELISA to determine antibody titer.

Particularly, a 96-well microplate was coated with recombinant S-protein (Dobeel Corp., Korea) at the concentration of 100 ng/well and blocked by adding 1% BSA for one hour. The microplate was washed and appropriately diluted serum was added to each well and incubated at 37°C for 2 hours. Then, Anti mouse goat IgG-HRP (Horse Radish Peroxidase; Sigma, USA) as a secondary antibody was added to each well and incubated at 37°C for one hour. At the end of incubation plates were washed extensively with PBST (PBS with Tween 20), and TMB (3, 3', 5, 5'-tetramethyl benzidine) peroxidase substrate solution (KPL, USA) was added, and followed by incubation at room temperature for 20 minutes. Then, OD₄₅₀ was measured with an ELISA reader. Antibody titer was determined as the inverse value of antibody final dilution to give OD reading that is three times high OD of the negative control.

As shown in Table 1 and Figure 1, use of Pam3Cys-SKKKK or poly I:C was more effective than aluminum hydroxide in inducing higher antibody titer against S-protein of HBV envelop protein. Specially, when the mixture of Pam3Cys-SKKKK and poly I:C was used, the induced antibody titer was slightly higher than the combined value of individually induced antibody titer indicating synergistic effect of these two components. The synergistic effect of combined use of Pam3Cys-SKKKK and Poly I:C is more pronounced in the induction of Pre S antibody. This could be due to the amount of antigen used, since the amount of pre S in L-HBsAg preparation is less than 10% of the total. And the 0.5 ug of antigen is near the saturating amount for immune response in mice (data not presented).

### <1-2-2> Antibody titer against preS

Antibody titer was determined by the same way as described in Example <1-2-1> except that preS antigen (Dobeel Corp., Korea) was used as an antibody capturing antigen.

As shown in Table 1 and Figure 2, the adjuvant mixture containing both Pam3Cys-SKKKK and poly I:C was more effective in inducing higher antibody titer against preS. Induction of pre S antibody by the adjuvant mixture was synergistic, inducing more than 4 times of pre S antibody compare to the added value of pre S antibody titer induced by Pam3Cys-SKKKK alone and poly I:C alone.

### <1-2-3> Isotypes of induced HBsAg specific antibody

Antibody titer was determined by the same manner as described in Example <1-2-1> except IgG1, IgG2a and IgG2b (mouse monoclonal antibody isotyping reagents; Sigma, USA) were used as a secondary antibody. And IgG2a/IgG1 and IgG2b/IgG1 ratios were also calculated by using the obtained antibody titer.

As shown in Figure 3a, isotypes IgG2a and IgG2b were much higher with the adjuvant mixture comparing to the values obtained with aluminum hydroxide. Specially, the induction of IgG2b was more than 20 times of the value obtained with aluminum hydroxide. When the adjuvant mixture comprising Pam3Cys-SKKKK and poly I:C was used, IgG2a/IgG1 ratio was about 10 times higher than aluminum hydroxide. Specially, the production of IgG2b was significantly high, and IgG2b/IgG1 ratio was much higher than IgG2a/IgG1 (Figure 3b and Figure 3c).

### Example 2: Stimulation of immunogenicity of influenza virus antigen

Influenza virus antigen was formulated with aluminum hydroxide, Pam3Cys-SKKKK alone, poly I:C alone, or with the mixture of Pam3Cys-SKKKK and Poly I:C as adjuvants and induced antibody titer was determined using influenza virus antigen as capturing antigen as for HBsAg antibody assay.

### <2-1> Formulation of Influenza virus antigen and administration

Different formulations were prepared by mixing recombinant split vaccine antigen (Korea Vaccine Co., Ltd, Korea) and the said adjuvant, which were administered to mice. The antigen was prepared by infecting the allantoic sac of a developing egg with influenza virus strains A/New Caledonia/20/99(H1N1), a/Wisconsin/67/2005(H3N2) and B/Malaysia/2506/2004, culturing, purifying and inactivating thereof.

Particularly, as shown in Table 2, 20 µg of Pam3Cys-SKKKK alone, poly I:C alone, the mixture of Pam3Cys-SKKK and Poly I:C, or aluminum hydroxide were mixed with 1.8 µg of the split vaccine antigen to give various different vaccine formulations in oil emulsion form. These different formulations were given by intramuscular injection to 5 week old C57BL/6 female mice two times at three weeks interval. The negative control received only PBS, while the positive control received the said antigen alone without any adjuvant. All group contains 6 mice.

**Table 2**

| | Negative control | Positive control 1 | Positive control 2 | Experiment al group 1 | Experiment al group 2 | Experiment al group 3 |
|---|---|---|---|---|---|---|
| Adjuvant | - | - | Aluminum hydroxide | Pam3Cys-S KKKK | Poly I:C | Pam3Cys-S KKKK + poly I:C |
| HA antibody titer | - | 2.1×10⁵ | 9.3×10⁵ | 6.8×10⁵ | 8.6×10⁵ | 3.0×10⁶ |
| IgG2a/IgG1 (%) | - | 8.85 | 6.09 | 7.59 | 6.17 | 31.67 |

### <2-2> Analysis of immune response

Serum was collected from each mouse before the vaccine administration and 2 weeks after the second vaccine administration and antigen specific antibody generation was analyzed by ELISA to determine antibody titer.

### <2-2-1> Determination of HA antibody titer

Antibody titer was determined by the same manner as described in Example <1-2-1> except that HA antigen (Korea Vaccine Co., Ltd, Korea) was used as an antibody capturing antigen.

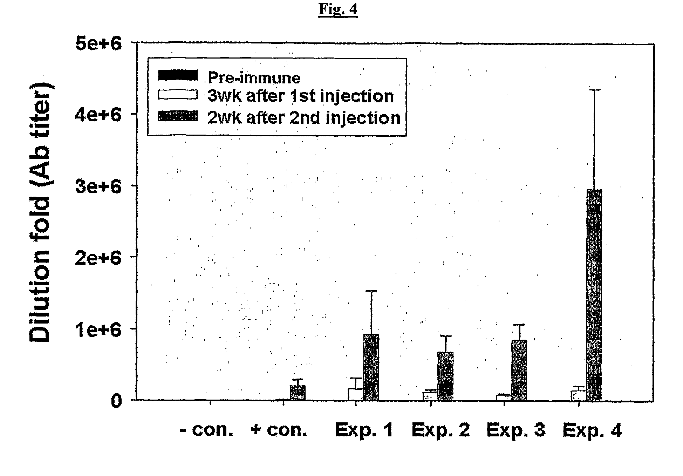

As shown in Table 2 and Figure 4, the mixture of Pam3Cys-SKKKK and poly I:C was more effective in inducing higher antibody titer against HA (synergistic effect) than single component or aluminum hydroxide.

### <2-2-2> Isotypes of induced HA specific antibody

Antibody titer was determined by the same way as described in Example <2-2-1> except that IgG1 and IgG2a were used as secondary antibodies for isotype determination. IgG2a/IgG1 and IgG2b/IgG1 ratios were also calculated by using the obtained antibody titer.

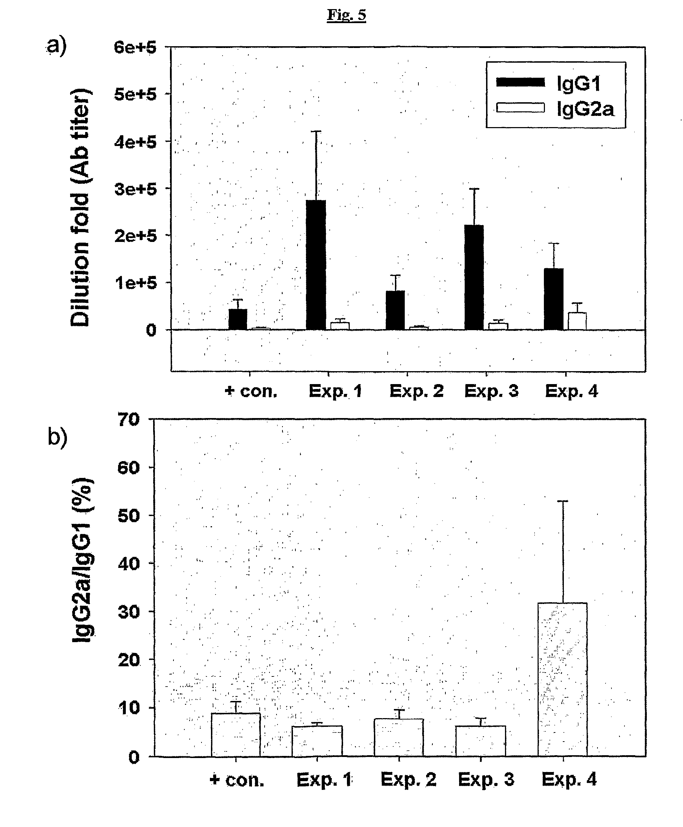

Figure 5a shows antibody titers of isotypes IgG1, and IgG2a. Isotype ratios were also calculated using the antibody titers. IgG2a/IgG1 value was specifically high (Figure 5b) with the adjuvant mixture compare to the value obtained with single components or Aluminum hydroxide.

### Example 3: Preparation of a powerful vaccine using recombinant HBsAg (S-protein from Hansenula polymorpha) and recombinant preS protein (from Saccharomyces cerevisiae)

Various vaccine formulations were prepared with recombinant HBsAg and recombinant preS protein by using aluminum hydroxide or the mixture of Pam3Cys-SKKKK and poly I:C as adjuvants, and immune responses were compared.

### <3-1> Preparation of vaccines and administration thereof

Vaccines were prepared by mixing recombinant HBsAg (Dobeel Corp., Korea), recombinant preS protein (Dobeel Corp., Korea) and said adjuvants, and they were administered by intra-muscular injection to mice. The recombinant HBsAg contained only S-protein without preS antigen and the recombinant preS protein prepared as a particle type by conjugating them to colloidal gold were used as antigens.

Particularly, a mixture containing 20 µg each of Pam3Cys-SKKKK and poly I:C was used as adjuvant with 0.5 µg of recombinant S-protein and 5 µg of preS protein to give a vaccine in oil emulsion form. Each test vaccine contained 0.5 µg of the recombinant S-protein and 5 µg of the preS per dose, which was then given by intramuscular injection to 5 week old C57BL/6 female mice three times at two weeks intervals.

Negative control is the group received PBS only. Positive control is the group received the mixture comprising aluminum hydroxide, S-protein, and colloidal gold conjugated recombinant preS antigen. Experimental group 1 is the group received vaccine prepared by mixing emulsified S-protein, Pam3Cys-SKKKK and poly I:C and colloidal gold conjugated recombinant preS antigen. Experimental group 2 is the group received vaccine prepared by emulsification of all components together, including S-protein, colloidal gold conjugated recombinant preS antigen and the mixture of Pam3Cys-SKKKK and poly I:C.

### <3-2> Analysis of immune responses

Serum samples were collected before the vaccine administration and 2 weeks after the third vaccine administration, and antigen specific antibody generation was analyzed by ELISA and expressed as antibody titer.

### <3-2-1> Antibody titer against S-protein

Antibody titer against S-protein was determined by the same way as described in Example <1-2-1>.

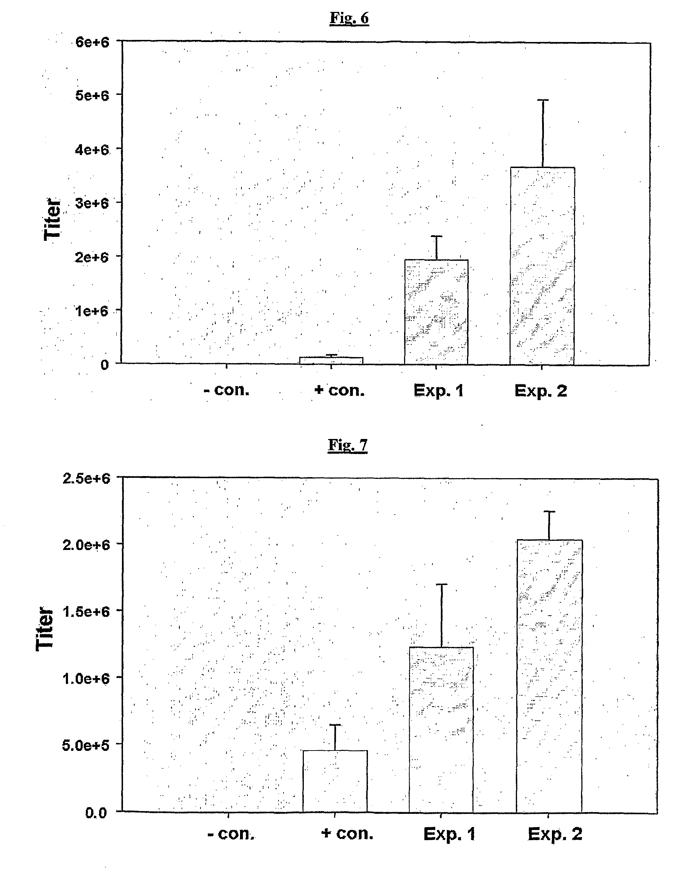

As shown in Table 3 and Figure 6, the adjuvant mixture of Pam3Cys-SKKKK and poly I:C induced more than 10 times higher antibody titer against S-protein than aluminum hydroxide. Particularly, the vaccine prepared by emulsifying all components together induced higher antibody titer than the vaccine prepared by emulsifying S-protein with the said adjuvant first, and adding the conjugated pre S.

**Table 3**

| | Negative | Positive control | Experimental | Experimental |
|---|---|---|---|---|
| | control | 1 | group 1 | group 2 |
| HBsAg antibody titer | - | 1.3×10⁵ | 1.9×10⁶ | 3.6×10⁶ |
| PreS antibody titer | - | 4.6×10⁵ | 1.2×10⁶ | 2.0×10⁶ |
| IgG2a/IgG1 (%) | - | 35 | 83 | 162 |
| IgG2b/IgG1 (%) | - | 6 | 110 | 324 |

### <3-2-2> Pre S antibody titer

Pre S antibody titer was determined by the same manner as described in Example <1-2-2>.

As shown in Table 3 and Figure 7, use of Pam3Cys-SKKKK and poly I:C as adjuvant mixture induced higher antibody titer against pre S compared to the use of aluminum hydroxide. Particularly, the vaccine prepared by emulsifying all components together-including HBsAg and preS antigen with the said adjuvant-induced pre S antibody most efficiently, inducing more than 3 times of the antibody by aluminum hydroxide.

### <3-2-3> Isotypes of induced HBsAg specific antibody

Isotype antibody titer was determined by the same method as described in Example <1-2-3>. And IgG2a/IgG1 and IgG2b/IgG1 ratios were calculated from the obtained antibody titer.

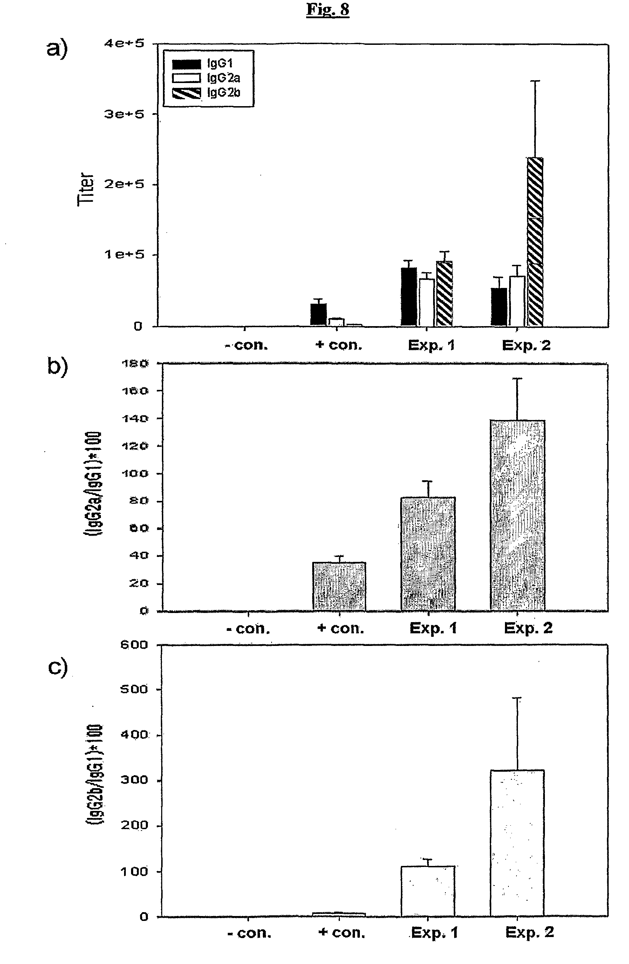

As shown in Figure 8a, antibody titers of isotypes IgG1, IgG2a and IgG2b were obtained. Isotype ratios were also calculated using the antibody titers. When the adjuvant mixture of Pam3Cys-SKKKK and poly I:C was used, IgG2a/IgG1 and IgG2b/IgG1 ratios were higher comparing to aluminum hydroxide. In particular, the vaccine, prepared by mixing HBsAg and preS antigen with the said adjuvant and emulsifying together, was confirmed more effective than the vaccine prepared by mixing preS antigen with emulsified HBsAg and the said adjuvant (Figures 8b and 8c).

### Example 4: Adjuvant composition using various lipopeptides

To test the synergistic adjuvant effect of various lipopeptides with poly I:C, Pam3Cys-SKKKK, Pam3Cys-SR8, or FSL-1 (Fibroblast-stimulating lipopeptide) is formulated with hepatitis B virus antigen.

### <4-1> Preparation of vaccines and administration

Vaccines were prepared by mixing hepatitis B virus whole surface antigen that was preadsorbed on aluminum hydroxide and said adjuvant components, and they were administered to mice. In the course of experiments, it was noticed that the aluminum hydroxide adsorbed antigen is more stable, and the aluminum hydroxide has no noticeable effect on the adjuvant effect of lipopeptide and poly I:C mixture (data not presented).

Particularly, as shown in Table 4, 0.5 µg of L-HBsAg absorbed to aluminum hydroxide was formulated with 20 µg of each of the lipopeptides or poly I:C (experimental group 1-4). Also the same amount of antigen was formulated with the mixture of lipopeptide and poly I:C (experimental group 5-7).

Vaccines were injected intra muscularly into 6 weeks old C57BL/6 female mice three times at two weeks intervals. The negative control was administered only with PBS without the vaccine and the antigen, while the positive control was administered with the said antigen formulated with aluminum hydroxide.

**Table 4**

| | Adjuvant | HBsAg antibody titer | preS antibody titer | IgG2a/Ig G1(%) | IgG2b/IgG 1 (%) |
|---|---|---|---|---|---|
| Negative control | - | - | - | - | - |
| Positive control | Aluminum hydroxide | 5.9×10⁵ | 1.6×10⁵ | 3.6 | 21.6 |
| Experimental group 1 | Pam3Cys-SKK KK | 6.5×10⁵ | 1.3×10⁵ | 17.4 | 159.4 |
| Experimental group 2 | Pam3Cys-SR8 | 4.8×10⁵ | 1.8×10² | 34.3 | 550.03 |
| Experimental group 3 | FSL-1 | 4.4×10⁵ | 1.4×10⁵ | 9.3 | 122.5 |
| Experimental group 4 | Poly I:C | 6.3×10⁵ | 2.3×10⁵ | 8.04 | 89.05 |
| Experimental group 5 | Pam3Cys-SKK KK + Poly I:C | 3.0×10⁶ | 1.4×10⁶ | 63.7 | 805.6 |
| Experimental group 6 | Pam3Cys-SR8 + Poly I:C | 1.7×10⁶ | 1.5×10⁶ | 50.4 | 306.2 |
| Experimental group 7 | FSL-1 + Poly I:C | 9.8×10⁵ | 6.4×10⁵ | 23.9 | 510.5 |

### <4-2> Analysis of immune response

Serum was collected from each mouse before the vaccine administration and 2 weeks after the last vaccine administration and antigen specific antibody generation was analyzed by ELISA to determine antibody titer.

### <4-2-1> Antibody titer against HBsAg (S-protein)

Antibody titer was determined by the same method as described in Example <1-2-1>.

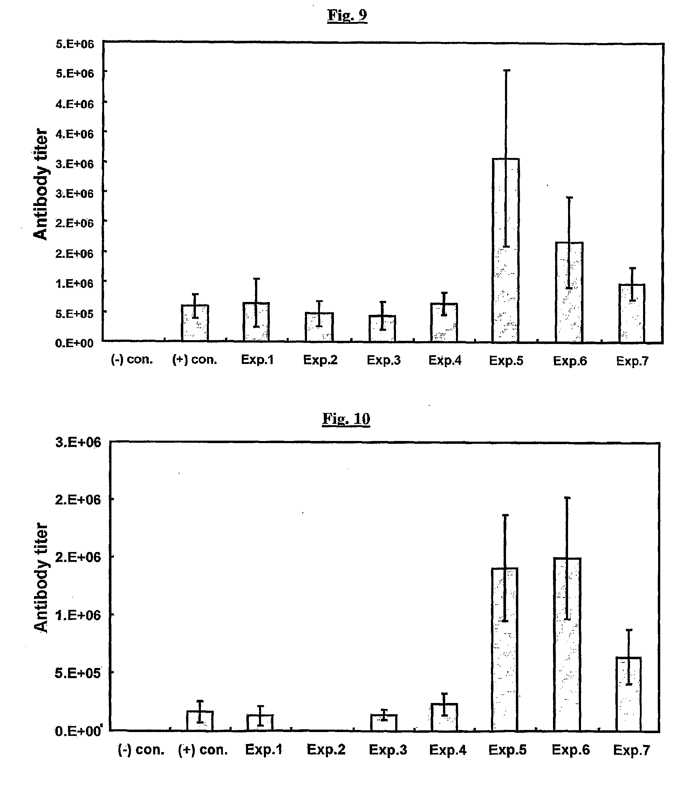

As shown in Table 4 and Figure 9, when the mixture of the lipopeptide (such as Pam3Cys-SKKKK and Pam3Cys-SR8) and poly I:C was used, the induced antibody titer was slightly higher than the combined value of individually induced antibody titer indicating synergistic effect of these two components.

### <4-2-2> Antibody titer against preS

Antibody titer was determined by the same way as described in Example <1-2-1> except that preS antigen (Dobeel Corp., Korea) was used as an antibody capturing antigen.

As shown 1 Table 4 and Figure 10, the adjuvant mixture containing one of the lipopeptides and poly I:C was more effective in inducing higher antibody titer against preS. The synergistic effect of combined use of lipopeptides other than Pam3Cys-SKKKK and poly I:C is also more dramatic for pre S antibody generation as seen before with Pam3Cys-SKKKK and poly I:C in the example <1-2-2>.

### <4-2-3> Isotypes of induced HBsAg specific antibody

Antibody titer was determined by the same manner as described in Example <1-2-1> except IgG1, IgG2a and IgG2b were used as a secondary antibody. And IgG2a/IgG1 and IgG2b/IgG1 ratios were also calculated by using the obtained antibody titer.

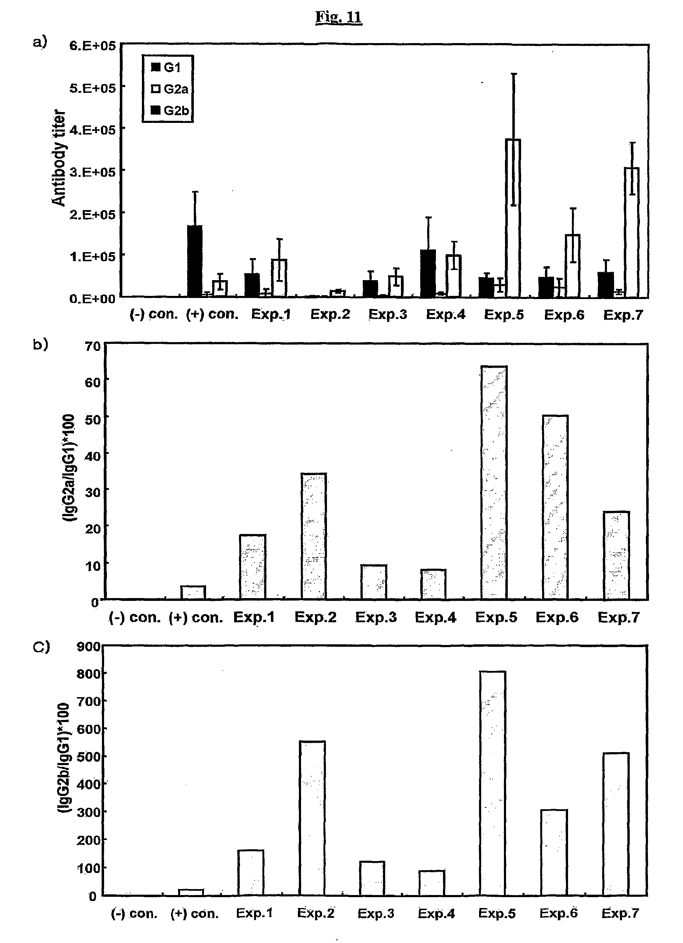

As shown in Figure 11a, antibody titers of isotypes IgG2a and IgG2b were much higher when the combination of the lipopeptide and poly I:C was used as an adjuvant compared to the value induced with aluminum hydroxide as adjuvant. Consequently the IgG2a/IgG1 and IgG2b/IgG1 ratio were also higher when the adjuvant mixture was used (Figure 11b, c). The synergistic effects were similar when the Pam3Cys-SKKKK, Pam3Cys-SR8 or FLS-1 was used as the lipopeptide component. Specially the combination of Pam3Cys-SKKKK and poly I:C was the most effective.

## Claims

1. A vaccine composition comprising:
- one or more lipopeptide selected from the group consisting of Pam3Cys-SKKKK and Pam3Cys-SR8;
- polyI:C (polyinosinic: polycytidylic acid) and
- at least one viral antigen or parasite antigen;
for use in the treatment of viral or parasitic infection.

2. The vaccine composition according to claim 1, wherein the antigen is selected from the group consisting of L-HBsAg, influenza HA, S-protein, and preS.

3. The vaccine composition according to claim 1, which is capable of efficiently inducing cell mediated immunity and producing appropriate antigen-specific high quality antibodies, including IgG1, IgG2a and IgG2b.

4. A therapeutic agent comprising:
- one or more lipopeptide selected from the group consisting of Pam3Cys-SKKKK and Pam3Cys-SR8;
- poly I:C(polyinosinic: polycytidylic acid) and
- at least one viral antigen or parasite antigen;
for use in the treatment of viral or parasitic infection.

5. The therapeutic agent according to claim 4, wherein the viral antigen is influenza virus antigen (HA: haemagglutinin or neuraminidase antigen), human papilloma virus (HPV) antigen, human immunodeficiency virus (HIV) antigen (GP-120, GP-160), vesicular stomatitis virus antigen (vesicular stomatitis virus glycoprotein), cytomegalovirus (CMV) antigen, hepatitis antigen [hepatitis A(HAV), B(HBV), C(HCV), D(HDV) and G(HGV): L-HBsAg, S-HBsAg, M-HBsAg, pre S], respiratory synctytial virus (RSV) antigen or herpes simplex virus antigen.

6. An immunological agent comprising:
- one or more lipopeptide selected from the group consisting of Pam3Cys-SKKKK and Pam3Cys-SR8;
- polyI:C (polyinosinic: polycytidylic acid) and
- a least one cancer-specific antigen;
for use in the prevention or the treatment of cancer.

7. The immunological agent according to claim 6, wherein the cancer is renal cell carcinoma, a melanoma, a chronic lymphocytic leukemia, a lung cancer, a cervical cancer, a stomach cancer, a thyroid cancer, a pancreatic cancer, a breast cancer, a prostate cancer, an ovarian cancer, a cholangioma, a liver cancer, a colon cancer, or a rectal cancer.

8. The immunological agent according to claim 6, wherein the cancer-specific antigen is gp100, MART-1 and MAGE-1, tyrosinase, CEA (cancer embryonic antigen), PSA (prostate specific antigen), HER2/neu, MAGE-1, MAGE-2, MAGE-3, NY-ESO-1, MUC-1, START-1 or SART-3, TERT (telomerase reverse transcriptase) or a partial peptide derived from TERT, WT1 or a partial peptide derived from WT1, Survivin-2B or a partial peptide derived from Survivin-2B, gp75, MDM2, telomerase, alpha-1 fetoprotein, CA125, CA15-3, CA19-9, G250 or NY-ESO-1.

## Patentansprüche

1. Impfstoffzusammensetzung umfassend:
- ein oder mehrere aus der Gruppe bestehend aus Pam3Cys-SKKKK und Pam3Cys-SR8 ausgewählte Lipopeptide;
- Polyl:C (Polyinosinsäure : Polycytidylsäure) und
- mindestens ein Virusantigen oder Parasitenantigen;
zur Verwendung bei der Behandlung einer Virus- oder Parasiteninfektion.

2. Impfstoffzusammensetzung nach Anspruch 1, wobei das Antigen aus der Gruppe bestehend aus L-HBsAg, Influenza HA, S-Protein und preS ausgewählt ist.

3. Impfstoffzusammensetzung nach Anspruch 1, welche in der Lage ist, zellvermittelte Immunität wirksam herbeizuführen und geeignete antigenspezifische hochwertige Antikörper einschließlich IgG1, IgG2a und IgG2b zu bilden.

4. Therapeutikum umfassend:
- ein oder mehrere aus der Gruppe bestehend aus Pam3Cys-SKKKK und Pam3Cys-SR8 ausgewählte Lipopeptide;
- Polyl:C (Polyinosinsäure : Polycytidylsäure) und
- mindestens ein Virusantigen oder Parasitenantigen;
zur Verwendung bei der Behandlung einer Virus- oder Parasiteninfektion.

5. Therapeutikum nach Anspruch 4, wobei das Virusantigen das Influenzavirus-Antigen (HA: Hämagglutinin- oder Neuraminidase-Antigen), Humanpapillomavirus-Antigen (HPV-Antigen), Humanimmundefizienzvirus-Antigen (HIV-Antigen) (GP-120, GP-160), Antigen des Virus der vesikulären Stomatitis (Glykoprotein des Virus der vesikulären Stomatitis), Zytomegalievirus-Antigen (ZMV-Antigen), Hepatitis-Antigen [Hepatitis A (HAV), B (HBV), C (HCV), D (HDV) und G (HGV) : L-HBsAg, S-HBsAg, M-HBsAg, preS], Respiratory-Syncytial-Virus-Antigen (RSV-Antigen) oder Herpes-simplex-Virus-Antigen ist.

6. Immunologisches Mittel umfassend:
- ein oder mehrere aus der Gruppe bestehend aus Pam3Cys-SKKKK und Pam3Cys-SR8 ausgewählte Lipopeptide;
- Polyl:C (Polyinosinsäure : Polycytidylsäure) und
- mindestens ein krebsspezifisches Antigen;
zur Verwendung bei der Vorbeugung oder der Behandlung von Krebs.

7. Immunologisches Mittel nach Anspruch 6, wobei der Krebs Nierenzellenkarzinom, ein Melanom, eine chronische lymphozytische Leukämie, ein Lungenkrebs, ein Gebärmutterhalskrebs, ein Magenkrebs, ein Schilddrüsenkrebs, ein Bauchspeicheldrüsenkrebs, ein Brustkrebs, ein Prostatakrebs, ein Eierstockkrebs, ein Cholangiom, ein Leberkrebs, ein Dickdarmkrebs oder ein Mastdarmkrebs ist.

8. Immunologisches Mittel nach Anspruch 6, wobei das krebsspezifische Antigen gp100, MART-1 und MAGE-1, Tyrosinase, CEA (karzinoembryonales Antigen), PSA (prostataspezifisches Antigen), HER2/neu, MAGE-1, MAGE-2, MAGE-3, NY-ESO-1, MUC-1, SART-1 oder SART-3, TERT (Telomerase-Reverse-Transkriptase) oder ein von TERT stammendes Teilpeptid, WT1 oder ein von WT1 stammendes Teilpeptid, Survivin-2B oder ein von Survivin-2B stammendes Teilpeptid, gp75, MDM2, Telomerase, Alpha-1-Fetoprotein, CA125, CA15-3, CA19-9, G250 oder NY-ESO-1 ist.

## Revendications

1. Composition vaccinale comprenant :
- un ou plusieurs lipopeptide(s) sélectionné(s) parmi le groupe constitué de Pam3Cys-SKKKK et Pam3Cys-SR8 ;
- du polyl:C (acide polyinosinique:polycytidylique) et
- au moins un antigène viral ou antigène parasitaire ;
pour l'utilisation dans le traitement d'infection virale ou parasitaire.

2. Composition vaccinale selon la revendication 1, dans laquelle l'antigène est sélectionné parmi le groupe constitué du L-HBsAg, du HA de la grippe, de la protéine S, et de la préS.

3. Composition vaccinale selon la revendication 1, qui est capable d'induire efficacement l'immunité à médiation cellulaire et de produire des anticorps de haute qualité spécifiques de l'antigène approprié, incluant IgG1, IgG2a et IgG2b.

4. Agent thérapeutique comprenant :
- un ou plusieurs lipopeptide(s) sélectionné(s) parmi le groupe constitué de Pam3Cys-SKKKK et Pam3Cys-SR8 ;
- du polyl:C (acide polyinosinique:polycytidylique) et
- au moins un antigène viral ou antigène parasitaire ;
pour l'utilisation dans le traitement d'une infection virale ou parasitaire.

5. Agent thérapeutique selon la revendication 4, dans lequel l'antigène viral est l'antigène du virus de la grippe (HA : antigène haemagglutinine ou neuraminidase), l'antigène du virus du papillome humain (VPH), l'antigène du virus de l'immunodéficience acquise humaine (VIH) (GP-120, GP-160), l'antigène du virus de la stomatite vésiculaire (glycoprotéine du virus de la stomatite vésiculaire), l'antigène du cytomégalovirus (CMV), l'antigène de l'hépatite [hépatite A (VHA), B (VHB), C (VHC), D (VHD) et G (VHG) : L-HBsAg, S-HBsAg, M-HBsAg, préS], l'antigène du virus syncytial respiratoire (VSR) ou l'antigène du virus de l'*herpès simplex.*

6. Agent immunologique comprenant :
- un ou plusieurs lipopeptide(s) sélectionné(s) parmi le groupe constitué de Pam3Cys-SKKKK et Pam3Cys-SR8 ;
- du polyl:C (acide polyinosinique:polycytidylique) et
- au moins un antigène spécifique du cancer ;
pour l'utilisation dans la prévention ou le traitement du cancer.

7. Agent immunologique selon la revendication 6, dans lequel le cancer est le carcinome des cellules rénales, un mélanome, une leucémie lymphoïde chronique, un cancer du poumon, un cancer du col de l'utérus, un cancer de l'estomac, un cancer de la thyroïde, un cancer du pancréas, un cancer du sein, un cancer de la prostate, un cancer de l'ovaire, un cholangiome, un cancer du foie, un cancer du côlon, ou un cancer du rectum.

8. Agent immunologique selon la revendication 6, dans lequel l'antigène spécifique du cancer est la gp100, MART-1 et MAGE-1, la tyrosinase, le CEA (antigène carcino-embryonnaire), le PSA (antigène spécifique de la prostate), HER2/neu, MAGE-1, MAGE-2, MAGE-3, NY-ESO-1, MUC-1, SART-1 ou SART-3, TERT (transcriptase inverse de la télomérase) ou un peptide partiel dérivé de la TERT, WT1 ou un peptide partiel dérivé de WT1, la Survivine-2B ou un peptide partiel dérivé de la Survivine-2B, la gp75, MDM2, la télomérase, la fétoprotéine alpha-1, CA125, CA15-3, CA19-9, G250 ou NY-ESO-1.
